# EUROPÄISCHE PATENTANMELDUNG

(11) **EP 1 048 305 A2**
(43) Veröffentlichungstag der Anmeldung: **02.11.2000**
(21) Anmeldenummer: 00890079.7
(22) Anmeldetag: 15.03.2000
(51) Int. Cl.: A61M 1/00, A61M 5/00

(54) **Anordnung zum Umfüllen von Blut**

(30) Priorität: 30.04.1999 AT 78299
(71) Anmelder: Österreichische Rote Kreuz, 1041 Wien (AT)
(72) Erfinder: Bertotti, Helga, 1050 Wien (AT); Werfring, Josef, 7223 Sieggraben (AT)
(74) Vertreter: Pinter, Rudolf, Dipl.-Ing.

(57) **Zusammenfassung**

Die Abpreßstation (10) einer Anordnung zum Umfüllen von in einem Abnahmebeutel (1) vorliegendem Spenderblut in einen Auffangbeutel (2) weist eine mechanische Abpreßeinheit (11) auf, in welcher der gegenüber dem Abnahmebeutel (1) tiefer angeordnete Auffangbeutel (2) zwischen mittels eines Preßantriebes (12) relativ zueinander bewegbaren Preßelementen (13) zusammendrückbar ist. Damit kann das Auspressen von Luft bzw. Luftbläschen enthaltendem Blut nach dem Abschluß des unter Wirkung der Schwerkraft erfolgenden Umfüllens und Filtrierens aus dem Auffangbeutel (2) mechanisch und standardisiert erfolgen, was die Bedienung der Anordnung vereinfacht und die Sicherheit erhöht.

## Beschreibung

Die Erfindung betrifft eine Anordnung zum Umfüllen von in einem Abnahmebeutel vorliegendem Spenderblut oder einer ähnlichen Flüssigkeit in einen Auffangbeutel, mit einer gegenüber dem Auffangbeutel erhöht angeordneten Trageinrichtung für den Abnahmebeutel, einer zumindest ein Filterelement samt mittels eines Rückschlagelementes nur in Richtung vom Auffangbeutel zum Abnahmebeutel durchströmbarem Bypass aufweisenden Verbindungsleitung zwischen Abnahmebeutel und Auffangbeutel und einer Abpreßstation für den Auffangbeutel, mittels welcher der Inhalt des Auffangbeutels über das Rückschlagelement teilweise wieder in die Verbindungsleitung zum Abnahmebeutel rückpressbar ist.

Das nach der Blutabnahme zumeist in aus durchsichtigem Kunststoff bestehenden Abnahmebeuteln vorliegende Spenderblut muß nach heutigen gesundheitlichen Standards vor der weiteren Bearbeitung vom Abnahmebeutel möglichst schonend umgefüllt und dabei über ein Filterelement - zumeist sogenannte Leukozytenfilter oder dergleichen - geführt werden. Da dabei insbesonders Augenmerk darauf zu richten ist, daß das im Auffangbeutel wiederum zwischengelagerte Blut keine Luft oder Luftbläschen enthält, sind bei diesem Umfüllen zumeist bestimmte Prozeduren vorgeschrieben, wie sie mit einer Anordnung der eingangs beschriebenen Art standardisiert durchführbar sind. Der Abnahmebeutel wird an der gegenüber dem Auffangbeutel erhöht angeordneten Trageinrichtung so aufgehängt, daß die wiederum als durchsichtiger Kunststoffschlauch ausgeführte Verbindungsleitung zum Auffangbeutel an seiner Unterseite austritt, sodaß das Umfüllen prinzipiell - nach Öffnen eventueller Absperrelemente an den Beuteln bzw. in der Verbindungsleitung - alleine unter der Wirkung der Schwerkraft erfolgen kann. Nachdem das Blut über die Verbindungsleitung und das Filterelement vollständig vom Abnahmebeutel in den Auffangbeutel übergelaufen ist, wird zur Entfernung allfälliger Luft oder Luftblasen, die sich unmittelbar unterhalb der oben in den Auffangbeutel geführten Verbindungsleitung sammeln, der Auffangbeutel händisch auf einer geeigneten Unterlage zusammengedrückt, womit vorerst Luft und Luftblasen und dann luftfreies Blut aus dem Auffangbeutel wieder zurück in die Verbindungsleitung zum Abnahmebeutel gepreßt werden. Um ein Rückspülen der erwähnten Filterelemente auszuschließen, sind diese entweder nur in der ursprünglichen Umfüllrichtung (also vom Abnahmebeutel zum Auffangbeutel) durchströmbar oder es ist auf der Ausströmseite ein Absperrorgan vorgesehen, welches vor dem beschriebenen Rückpressen zu schließen ist. Weiters ist ein Bypass zum Filterelement in der Verbindungsleitung vorgesehen, der dieses Rückströmen des Blutes zum Abnahmebeutel hin ermöglicht. Die aufsteigende Blutsäule wird solange in Richtung zum Abnahmebeutel hin zurückgepreßt, bis dort reines bzw. keine eingeschlossenen Luftblasen mehr aufweisendes Blut wieder in den Abnahmebeutel eintritt. Je nach Anforderungen kann dann der luftfreie Auffangbeutel verschlossen und entnommen oder der sonstigen weiteren Behandlung zugeführt werden - es ist aber auch möglich, das nun in der Verbindungsleitung befindliche (vorausgesetzt luftfreie) Blut zumindest zum Teil nochmals in den Auffangbeutel laufen zu lassen, um eine möglichst hohe Blutrückgewinnung zu erreichen. Der leere Abnahmebeutel samt Verbindungsleitung und Filterelement mit Bypass wird anschließend entsorgt.

Bei derartigen bekannten Anordnungen nachteilig ist insbesonders der Umstand, daß die händische Manipulation und insbesonders die beschriebene manuelle Luftentfernung einerseits bei größeren Mengen an zu behandelnden Abnahmebeuteln relativ anstrengend ist und daß andererseits mit einer derartigen manuellen Behandlung immer gewisse Fehlerquellen gegeben sind, die beispielsweise bei verbleibender Luft im Auffangbeutel zu einem Qualitätsverlust bzw. zu einem Lagerschaden des darin gespeicherten Blutes führen.

Aufgabe der vorliegenden Erfindung ist es, die beschriebenen Nachteile der bekannten Anordnungen der eingangs genannten Art zu vermeiden und insbesonders eine derartige Anordnung so zu verbessern, daß die beschriebene Behandlung weiter standardisiert werden kann und mögliche Fehlerquellen weiter ausgeschaltet werden können.

Diese Aufgabe wird gemäß der vorliegenden Erfindung bei einer Anordnung der eingangs genannten Art dadurch gelöst, daß die Abpreßstation eine mechanische Abpreßeinheit aufweist, in welcher der Auffangbeutel zwischen mittels eines Preßantriebes relativ zueinander bewegbaren Preßelementen zusammendrückbar ist. Abgesehen von den im Hinblick auf mögliche Lufteinschlüsse relativ unkritischen Manipulationen des Aufhängens der Abnahmebeutel bzw. des entsprechenden Anordnens der zugehörigen Auffangbeutel samt Verbindungsleitungen und beschriebener Elemente ist damit eine weitgehende Automatisierung insbesonders der beschriebenen Maßnahmen zum Luftaustreiben aus dem Auffangbeutel möglich. Das Abpressen des Auffangbeutels kann weitgehend automatisch über die Abpreßeinheit erfolgen, womit die mit dem Umfüllen befaßten Personen auch weit weniger anstrengende Handhabungen vornehmen müssen.

Als Preßelemente sind in bevorzugter Ausgestaltung der Erfindung zwei, vorzugsweise der Form des Auffangbeutels angepaßte, im wesentlichen flächig ausgebildete Preßplatten vorgesehen, von denen die eine in der Abpreßeinheit feststehend und die andere mittels des Preßantriebes bewegbar, vorzugsweise um eine seitliche Achse schwenkbar, ist. Dies ergibt eine sehr einfache Vorrichtung, die auch unkompliziert mit den Auffangbeuteln zu beschicken ist, wobei bedarfsweise ohne weiteres noch eine Sichtkontrolle der ablaufenden Vorgänge möglich ist.

Der Preßantrieb weist nach einer bevorzugten weiteren Ausgestaltung der Erfindung einen druckmittelbetätigbaren Arbeitszylinder, vorzugsweise einen Pneumatikzylinder, auf, der über einen seitlich angeordneten Schwenkhebel an der Schwenkachse der Preßplatte angreift. Derartige Antriebe sind kleinbauend, kraftvoll, leise und zuverlässig, was eine einfache Verwendung der erfindungsgemäßen Anordnung auch beispielsweise in Krankenhausumgebung oder bei gedrängten Platzverhältnissen ermöglicht.

Im Kraftweg vom Arbeitszylinder zur Preßplatte ist nach einer anderen vorteilhaften Weiterbildung der Erfindung ein Federspeicher-Element eingeschaltet, was auf sehr einfache und zuverlässige Weise z.B. Verletzungen des Bedienpersonals durch die zusammengedrückten Preßelemente verhindert, soferne nur diese Federspeicher-Elemente entsprechend ausgelegt sind.

Trageinrichtung und Abpreßstation sind in vorteilhafter Weiterbildung der Erfindung jeweils zur gleichzeitigen Aufnahme mehrerer Abnahme- und Auffangbeutel ausgebildet, wobei die Preßelemente und vorzugsweise auch der Preßantrieb jeweils für mehrere Auffangbeutel gemeinsam ausgelegt sind. Damit kann insbesonders das Umfüllen größerer Mengen von Spenderblut beschleunigt durchgeführt werden, wobei die Steuerung bzw. Überwachung der Funktion trotzdem von einer Bedienperson wahrgenommen werden kann.

In weiterer Ausgestaltung der Erfindung können die Preßplatten jeweils paarweise mit entsprechendem Abstand zum nächsten Plattenpaar auf einer Grundplatte der Abpreßstation hintereinander angeordnet sein, wobei die auf der Grundplatte gelagerten Schwenkachsen der bewegbaren Preßplatten in ein seitlich auf der Grundplatte vorgesehenes Gehäuse für den gemeinsamen Preßantrieb geführt sind. Damit ist eine relativ kleinbauende Anordnung mölich, die zu Folge des gemeinsamen Antriebes der bewegbaren Preßplatten auch sehr einfach aufgebaut ist.

Die Trageinrichtung für den (die) Abnahmebeutel kann nach einer anderen Weiterbildung der Erfindung einen relativ zur Abpreßstation höhenverstellbaren Liftteil aufweisen, was einerseits das Vorsehen verschiedener Fallhöhen für das umzufüllende bzw. zu filtrierende Blut ermöglicht und andererseits natürlich die von der Bedienperson beim Aufhängen der Abnahmebeutel vorzunehmenden Handhabungen in bequemer Höhe erlaubt und damit sehr vereinfacht. Im letztgenannten Zusammenhang besonders vorteilhaft ist eine weitere Ausgestaltung der Erfindung, gemäß welcher die Antriebe der Trageinrichtung und der Abpreßstation über eine gemeinsame Steuereinheit, vorzugsweise zumindest teilweise im Gehäuse für den Preßantrieb integriert, betätigbar sind. Mit dieser Steuerung können die erforderlichen Funktionsabläufe zumindest teilweise vorgegeben werden, sodaß die Bedienung einfacher und sicherer wird. Abgesehen von der im Hinblick auf Bedienung und Unterbringung vorteilhaften Integration im Gehäuse für den Preßantrieb könnte natürlich auch eine Steuerung über externe Einheiten (beispielsweise SPS oder dergleichen) vorgesehen werden.

In weiterer Ausgestaltung der Erfindung ist vorgesehen, daß die Abnahmebeutel auf der Trageinrichtung bzw. dem Liftteil mit der Öffnung an ihrem tiefsten Punkt und die Auffangbeutel in der Abpreßstation bzw. den Preßelementen mit der Öffnung an ihrem höchsten Punkt angeordnet sind, was auf sehr einfache Weise das eingangs bereits angesprochene Umfüllen oder Filtrieren unter alleiniger Wirkung der Schwerkraft ermöglicht, sodaß diesbezüglich keine weiteren Vorkehrungen zu treffen sind.

Im Bereich der Trageinrichtung und/oder der Abpreßstation können in weiterer Ausgestaltung der Erfindung Fluß- bzw. Füllstandsensoren für die Flüssigkeit bzw. das Blut angeordnet und mit der Steuereinheit bzw. einer Anzeige- und/oder Auswerteeinrichtung verbunden sein, was eine weitere Automatisierung des Umfüll- bzw. Filtrationsvorganges dadurch ermöglicht, daß die jeweils nächsten der beschriebenen Schritte immer von dem Ansprechen dieser Sensoren abhängig automatisch gesetzt werden können.

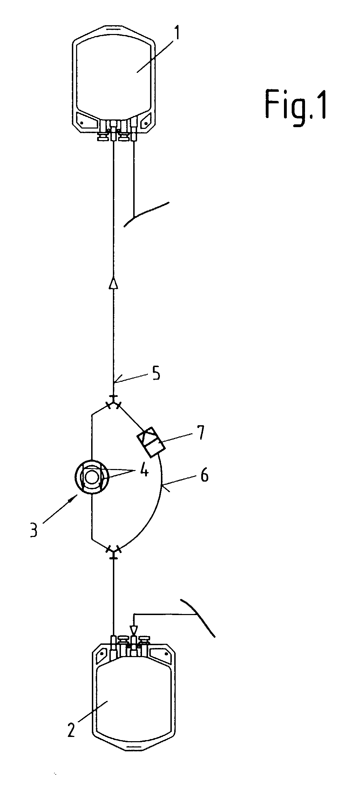

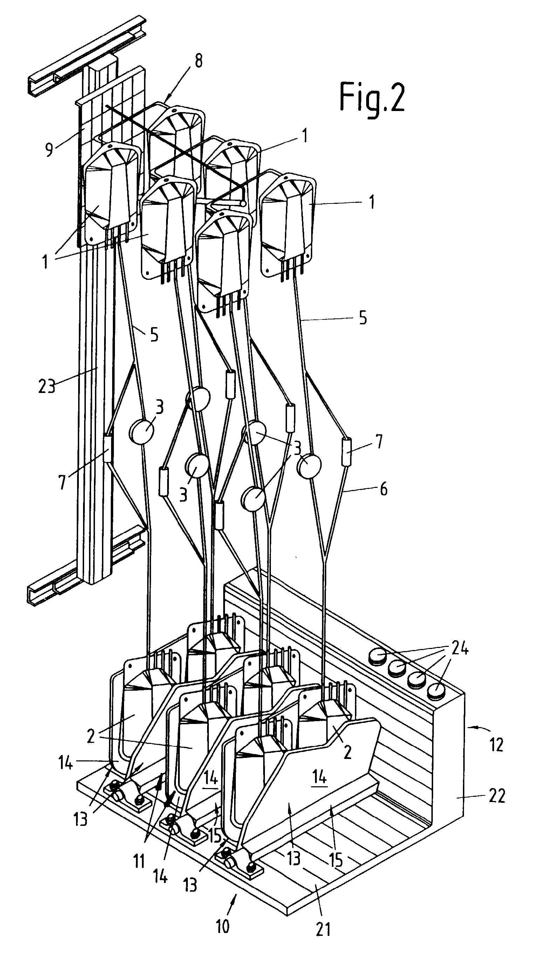

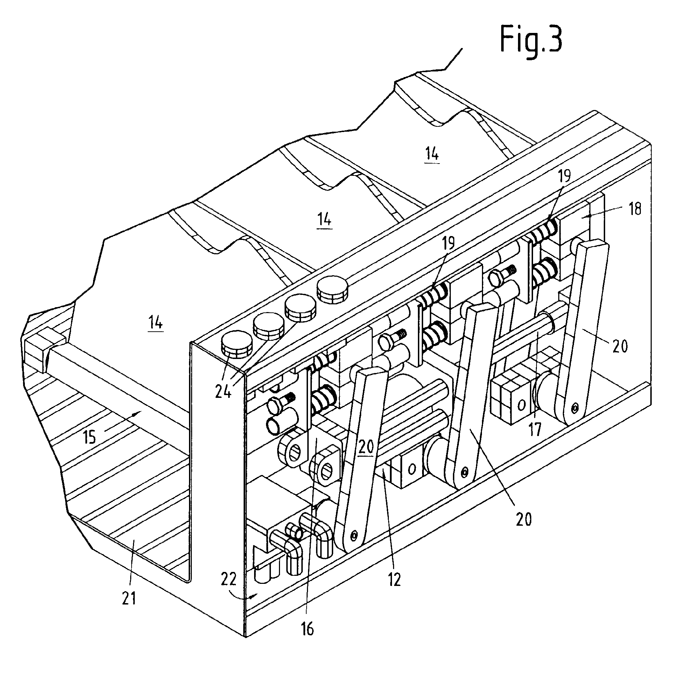

Die Erfindung wird im folgenden noch anhand der in der Zeichnung teilweise schematisch dargestellten Ausführungsbeispiele erläutert. Fig. 1 zeigt dabei eine grundsätzliche Anordnung der eingangs zum Stand der Technik beschriebenen Art, Fig. 2 zeigt eine Anordnung nach der vorliegenden Erfindung in perspektivischer Ansicht und Fig. 3 zeigt eine teilweise geöffnete seitliche Schrägansicht der Abpreßstation gemäß Fig.2.

Die Anordnung nach Fig. 1 dient zum Umfüllen von in einem Abnahmebeutel 1 vorliegendem Spenderblut in einen Auffangbeutel 2, wobei diese beiden Beutel 1, 2 hier identisch ausgebildet sind - davon abgesehen wäre aber natürlich auch eine unterschiedliche Ausbildung oder unterschiedliche Größe möglich, wobei dann beispielsweise auch ein größerer Abnahmebeutel mit zwei entsprechend kleineren Auffangbeuteln über Verbindungsverzweigungen verbunden sein könnte.

Diese Beutel 1, 2 bestehen üblicherweise aus durchsichtigem Kunststoff und haben diverse Anschlußöffnungen für verschiedenste hier nicht näher interessierende Zwecke der Weiterbehandlung ihres Inhaltes. Die hier interssierende und im folgenden beschriebene Anordnung kann mit verschiedensten Ausgestaltungen und Größen derartiger Beutel 1, 2 arbeiten, wobei lediglich die Auffangbeutel 2 aus im folgenden noch beschriebenen Gründen zumindest teilweise unter Verkleinerung ihres Innenvolumens zusammendrückbar sein müssen.

Der Abnahmebeutel 1 ist gegenüber dem Auffangbeutel 2 erhöht angeordnet und über eine ein Filterelement 3, beispielsweise einen nur in Richtung der eingezeichneten Pfeile 4 durchströmbaren Leukozytenfilter aufweisende Verbindungsleitung 5 mit dem Auffangbeutel 2 verbunden. Ob diese Verbindung im Sinne eines Beutelsystems fix und bereits von vornherein bestehend, nur zerstörend trennbar, oder aber steckbar oder dergleichen ist, ist hier belanglos. In einem Bypass 6 ist ein Rückschlagelement 7 angeordnet, welches nur in Richtung vom Auffangbeutel 2 zum Abnahmebeutel 1 (also entgegen der Pfeile 4) durchströmbar ist. Nicht dargestellt sind hier allfällige Absperreinrichtungen (Schlauchklemmen und dergleichen) in der Verbindungsleitung 5, die für die Handhabung unter Umständen notwendig oder vorteilhaft sind, jedoch nichts mit dem Wesentlichen der vorliegenden Erfindung zu tun haben.

Zum Umfüllen des im Abnahmebeutel 1 vorliegenden Spenderblutes in den Auffangbeutel 2 wird der Abnahmebeutel 1 in die dargestellte erhöhte Position gebracht, beispielsweise auf einer hier nicht dargestellten Trageinrichtung aufgehängt, wonach - allenfalls nach Öffnen der wie erwähnt hier nicht dargestellten Absperrelemente - das Blut unter der Wirkung der Schwerkraft vom Abnahmebeutel 1 über die Verbindungsleitung 5 und das Filterelement 3 in den Auffangbeutel 2 läuft. Es ist dabei nie ganz zu vermeiden, daß sich im Auffangbeutel nach dessen Vollaufen Luft bzw. Luftblasen unterhalb der Einmündung der Verbindungleitung 5 sammelt, was das Blut im Auffangbeutel 2 bei längerer Lagerung unbrauchbar machen bzw. dessen weitere Bearbeitung erschweren oder unmöglich machen würde. Es wird daher nach dem Vollaufen des Auffangbeutels 2 dieser zusammengepreßt, womit vorerst Luft und dann mit Luftblasen durchmischtes Blut zurück in die Verbindungsleitung 5 gedrückt wird, und zwar so lange, bis es über den Bypass 6 und das Rückschlagelement 7 wieder bis zum Abnahmebeutel 1 in der Verbindungsleitung 5 aufsteigt und bis im Abnahmebeutel 1 wieder blasenfreies Blut austritt. Dann kann der Auffangbeutel 2 am Anschluß der Verbindungsleitung 5 verschlossen und nach Abtrennen der Verbindungsleitung 5 entnommen werden oder aber das in der Verbindungsleitung 5 stehende, von Luftblasen freie Blut zumindest teilweise wieder rückfließen gelassen und erst dann der Auffangbeutel 2 verschlossen und entnommen bzw. dessen Inhalt weiter bearbeitet werden.

Der Abnahmebeutel 1 samt Verbindungsleitung 5 (mit Filterelement 3 und Bypass 6) wird anschließend entsorgt.

Gemäß Fig.2 ist nun einerseits eine Trageinrichtung 8 mit einem höhenverstellbaren Liftteil 9 für hier jeweils sechs Abnahmebeutel 1 und andererseits eine Abpreßstation 10 mit einer mechanischen Abpreßeinheit 11 vorgesehen, in welcher die hier sechs zugeordneten Auffangbeutel 2 zwischen mittels eines Preßantriebes 12 relativ zueinander bewegbaren Preßelementen 13 zusammendrückbar sind. Als Preßelemente 13 sind jeweils zwei, hier der Form von zwei Auffangbeuteln 2 angepaßte flächig ausgebildete Preßplatten 14 vorgesehen, von denen die eine, in der Darstellung hintere, in der Abpreßeinheit 11 feststehend und die andere, in der Darstellung vordere, mittels des Preßantriebes 12 um eine Achse 15 schwenkbar ist. Davon abgesehen, könnten als Preßelemente 13 auch andere geeignete Elemente, wie etwa parallele, relativ zueinander bewegbare Platten, Rollenkörper oder dergleichen vorgesehen werden, die mit einem entsprechendem Antrieb versehen ein Zusammendrücken der Auffangbeutel 2 erlauben.

Wie insbesonders in der Detaildarstellung nach Fig. 3 ersichtlich ist, weist der Preßantrieb 12 einen druckmittelbetätigbaren Arbeitszylinder 16, beispielsweise einen Pneumatikzylinder, auf, dessen Kolbenstange 17 auf einen Verstellschlitten 18 wirkt, welcher wiederum über Federspeicherelemente 19 auf seitlich angeordnete Schwenkhebel 20 einwirkt, die ihrerseits an den Schwenkachsen 15 der beweglichen Preßplatten 14 angreifen.

Die Preßplatten 14 sind jeweils paarweise mit entsprechendem Abstand zum nächsten Plattenpaar 14 auf einer Grundplatte 21 der Abpreßstation 10 hintereinander angeordnet, wobei die auf der Grundplatte 21 gelagerten Schwenkachsen 15 der bewegbaren Preßplatten 14 in das seitlich auf der Grundplatte 21 vorgesehene Gehäuse 22 für den gemeinsamen Preßantrieb 12 geführt sind. Damit ist eine sehr einfache mechanische Pressung der Auffangbeutel 2 möglich, wobei durch die beschriebenen Federspeicherelemente 19 im Kraftweg vom Arbeitszylinder 16 zur Preßplatte 14 sehr leicht sichergestellt werden kann, daß einerseits unbeabsichtigt zwischen die Preßplatten 14 kommende Fremdkörper keine Beschädigung des Antriebes verursachen und andererseits beispielsweise auch die Finger von Bedienpersonen nicht durch Quetschungen verletzt werden können.

Wie bereits angesprochen weist hier die Trageinrichtung 8 für die Abnahmebeutel 1 einen relativ zur Abpreßstation 10 höhenverstellbaren Liftteil 9 auf, der auf einer beispielsweise wandmontierten Schiene 23 gelangert und über hier nicht dargestellte Antriebselemente, (beispielsweise einen elektrisch oder pneumatischen Linearmotor, händisch unterstützte Seilzugeinrichtungen oder dergleichen) vertikal bewegbar ist. Die Antriebe der Trageinrichtung 8 und der Abpreßstation 10 können über eine gemeinsame Steuereinheit, welche hier zumindest teilweise im Gehäuse 22 für den Preßantrieb 12 integriert und durch die Bedienknöpfe 24 symbolisiert ist betätigbar sein. Im Rahmen der Erfindung könnte diese Steuerung aber natürlich auch separat bzw. über externe Einheiten, wie etwa speicherprogrammierbare Steuerungen (SPS) oder dergleichen erfolgen. Auch könnte an Stelle des pneumatischen Arbeitszylinders 16 natürlich auch jede andere geeignete Ausbildung des Preßantriebes vorgesehen werden, beispielsweise Hydraulikzylinder, elektrische Getriebemotoren, händisch unterstützte Hebelantriebe oder dergleichen.

Weiters könnten im Bereich der Trageinrichtung 8 und/oder der Abpreßstation 10 auch noch hier nicht dargestellte Fluß- bzw. Füllstandsensoren vorgesehen und mit einer Steuereinheit bzw. einer Anzeige- und/oder Auswerteeinrichtung oder dergleichen verbunden sein, was eine weitgehend automatisierte Umfüllung bzw. Filtration ermöglicht.

Die Arbeitsweise der in den Fig. 2 und 3 dargestellten Anordnung entspricht im wesentlichen der im Zusammenhang mit Fig. 1 obenstehend beschriebenen. Gemäß Fig. 2 kann der Liftteil 9 zum Aufhängen der Abnahmebeutel 1 in der Darstellung nach unten gefahren werden, sodaß die Bedienperson bequem diese Arbeit erledigen kann. Anschließend wird der Liftteil 9 mit den Abnahmebeuteln 1 samt angeschlossenen Verbindungsleitungen 5 hochgefahren und die Auffangbeutel 2 werden zwischen die Preßplatten 14 gelegt. Nach dem Überlaufen lassen des Inhaltes der Abnahmebeutel 1 erfolgt das beschriebene Abpressen der Auffangbeutel 2 zum Austreiben der Luft, wonach das Umfüllen wie beschrieben abgeschlossen wird.

Aus Fig. 2 ist auch ersichtlich, daß die Abpreßstation 10 ebenso wie die Trageinrichtung 8 zur gleichzeitigen Aufnahme weitgehend beliebig vieler Beutel 1, 2 augebildet werden kann, womit das Umfüllen und die Filtration größerer Blutmengen einfach und schnell auch von einer Bedienperson erledigt bzw. überwacht werden kann.

## Patentansprüche

1. Anordnung zum Umfüllen von in einem Abnahmebeutel (1) vorliegendem Spenderblut oder einer ähnlichen Flüssigkeit in einen Auffangbeutel (2), mit einer gegenüber dem Auffangbeutel (2) erhöht angeordneten Trageinrichtung (8) für den Abnahmebeutel (1), einer zumindest ein Filterelement (3) samt mittels eines Rückschlagelementes (7) nur in Richtung vom Auffangbeutel (2) zum Abnahmebeutel (1) durchströmbarem Bypass (6) aufweisenden Verbindungsleitung (5) zwischen Abnahmebeutel (1) und Auffangbeutel (2) und einer Abpreßstation (10) für den Auffangbeutel (2) mittels welcher der Inhalt des Auffangbeutels (2) über das Rückschlagelement (7) teilweise wieder in die Verbindungsleitung (5) zum Abnahmebeutel (1) rückpressbar ist, **dadurch gekennzeichnet,** daß die Abpreßstation (10) eine mechanische Abpreßeinheit (11) aufweist, in welcher der Auffangbeutel (2) zwischen mittels eines Preßantriebes (12) relativ zueinander bewegbaren Preßelementen (13) zusammendrückbar ist.

2. Anordnung nach Anspruch 1, dadurch gekennzeichnet, daß als Preßelemente (13) zwei, vorzugsweise der Form des Auffangbeutels (2) angepaßte, im wesentlichen flächig ausgebildete Preßplatten (14) vorgesehen sind, von denen die eine in der Abpreßeinheit (11) feststehend und die andere mittels des Preßantriebes (12) bewegbar, vorzugsweise um eine seitliche Achse (15) schwenkbar, ist.

3. Anordnung nach Anspruch 2, dadurch gekennzeichnet, daß der Preßantrieb (12) einen druckmittelbetätigbaren Arbeitszylinder (16), vorzugsweise einen Pneumatikzylinder, aufweist, der über einen seitlich angeordneten Schwenkhebel (20) an der Schwenkachse (15) der Preßplatte (14) angreift.

4. Anordnung nach Anspruch 3, dadurch gekennzeichnet, daß im Kraftweg vom Arbeitszylinder (16) zur Preßplatte (14) ein Federspeicher-Element (19) eingeschaltet ist.

5. Anordnung nach einem oder mehreren der Ansprüche 1 bis 4, dadurch gekennzeichnet, daß Trageinrichtung (8) und Abpreßstation (10) jeweils zur gleichzeitigen Aufnahme mehrerer Abnahme- und Auffangbeutel (1, 2) ausgebildet sind, wobei die Preßelemente (13) und vorzugsweise auch der Preßantrieb (12) jeweils für mehrere Auffangbeutel (2) gemeinsam ausgelegt sind.

6. Anordnung nach einem oder mehreren der Ansprüche 2 bis 5, dadurch gekennzeichnet, daß die Preßplatten (14) jeweils paarweise mit entsprechendem Abstand zum nächsten Plattenpaar auf einer Grundplatte (21) der Abpreßstation (10) hintereinander angeordnet sind, wobei die auf der Grundplatte (21) gelagerten Schwenkachsen (15) der bewegbaren Preßplatten (14) in ein seitlich auf der Grundplatte (21) vorgesehenes Gehäuse (22) für den gemeinsamen Preßantrieb (12) geführt sind.

7. Anordnung nach einem oder mehreren der Ansprüche 1 bis 6, dadurch gekennzeichnet, daß die Trageinrichtung (8) für den (die) Abnahmebeutel (1) einen relativ zur Abpreßstation (10) höhenverstellbaren Liftteil (9) aufweist.

8. Anordnung nach einem oder mehreren der Ansprüche 1 bis 7, dadurch gekennzeichnet, daß die Antriebe der Trageinrichtung (8) und der Abpreßstation (10) über eine gemeinsame Steuereinheit, vorzugsweise zumindest teilweise im Gehäuse (22) für den Preßantrieb (12) integriert, betätigbar sind.

9. Anordnung nach einem oder mehreren der Ansprüche 1 bis 8, dadurch gekennzeichnet, daß die Abnahmebeutel (1) auf der Trageinrichtung (8) bzw. dem Liftteil (9) mit der Öffnung an ihrem tiefsten Punkt und die Auffangbeutel (2) in der Abpreßstation (10) bzw. den Preßelementen (13) mit der Öffnung an ihrem höchsten Punkt angeordnet sind.

10. Anordnung nach einem oder mehreren der Ansprüche 1 bis 9, dadurch gekennzeichnet, daß im Bereich der Trageinrichtung (8) und/oder der Abpreßstation (10) Fluß- bzw. Füllstandsensoren für die Flüssigkeit angeordnet und mit der Steuereinheit bzw. einer Anzeige- und/oder Auswerteeinrichtung verbunden sind.
